# EUROPEAN PATENT APPLICATION

(11) **EP 1 867 344 A1**
(43) Date of publication of application: **19.12.2007**
(21) Application number: 07252435.8
(22) Date of filing: 14.06.2007
(51) Int. Cl.: A61L 2/07, A61L 2/24

(54) **Improved autoclave and sterilisation process**

(30) Priority: 15.06.2006 GB 0611817
(71) Applicant: Eschmann Holdings Limited, Eschmann House, Peter Road Lancing West Sussex, BN15 8TJ (GB)
(72) Inventor: Brake, Stephen John, West Sussex, BN15 8TJ (GB); Hainsworth, John Kenneth, Cambridge, CB4 0DW (GB)
(74) Representative: Peel, James Peter

(57) **Abstract**

According to the invention, a pre-conditioning method of operating an autoclave (10) during a pre-conditioning stage of a sterilisation cycle wherein the autoclave (10) has a chamber (20) having an external wall (30), a supply of steam (50), a heater (70) to heat the external wall (30) of the chamber (20) and vacuum pump (100), which comprises the steps of:
applying a first vacuum pulse in the chamber (20);
heating the chamber (20);
supplying steam to the chamber (20) at a pressure above atmospheric pressure for a holding period which is sufficiently long for the contents of the chamber (20) to be heated; and
applying a second vacuum pulse in the chamber (20); has been found to be effective as a pre-conditioning stage for a sterilisation cycle for sterilising a porous load.

## Description

The present invention provides an improved sterilisation process to be used in a vacuum-type autoclave.

Two types of sterilisation cycles are known: vacuum cycles and non-vacuum cycles. The non-vacuum cycle is generally only used to process a simple solid load having no packaging materials. No vacuum is required, since air can be removed from around the load with a simple steam flush.

The vacuum cycle is used to process a porous load with complex geometries (for example, a load containing an instrument with an internal lumen), a porous load (such as drapes or gowns) or a pre-packaged load (instruments sealed in a paper package ready for sterilisation). It is necessary to create a vacuum in the autoclave chamber in order to extract air from the porous load so that steam is able to penetrate to internal surfaces.

Both a non-vacuum cycle and a vacuum cycle comprise three stages which are preconditioning, sterilisation and drying. The purpose of the preconditioning stage is to heat up the chamber and the load, and to remove air from the chamber. The presence of air in the chamber impedes sterilisation. The sterilisation stage is performed by holding the load at a fixed, high temperature for a fixed period of time. The sterilisation stage of the cycle is generally defined by sterilisation standards. The load is dried in the drying stage and moisture is removed from any packaging materials or porous fabrics. If packaging materials are not sufficiently dry at the end of the cycle, there is a danger that the load can become re-contaminated. This is because pathogens can penetrate moist packaging. Each stage consists of a series of heating and pumping operations which are used to vary the pressure, temperature, and air content of the sterilisation chamber.

Users of autoclaves are demanding that the cycle time be reduced so that the sterilisation process is quicker. A way of ameliorating this problem has been sought.

According to the invention there is provided a pre-conditioning method of operating an autoclave during a pre-conditioning stage of a sterilisation cycle wherein the autoclave has a chamber, a supply of steam and a vacuum pump which method comprises the steps of
applying a first vacuum pulse in the chamber;
supplying steam to the chamber at a pressure above atmospheric pressure for a holding period which is sufficiently long for the contents of the chamber to be heated; and
applying a second vacuum pulse in the chamber.

According to the invention there is further provided a drying method of operating an autoclave to perform a drying stage of a sterilisation cycle wherein the autoclave has a chamber, a supply of steam and a vacuum pump which method comprises the steps of
applying a vacuum pulse;
supplying the chamber with air;
wherein the vacuum pulse is applied until the pressure in the chamber reaches a pre-set level.

According to the invention there is also provided a sterilisation method of operating an autoclave to perform a sterilisation cycle wherein the autoclave has a chamber, a supply of steam and a vacuum pump which method comprises the steps of
a pre-conditioning method according to the invention;
pressurising the chamber to a sterilisation plateau by supplying steam to the chamber;
maintaining the chamber at a pre-selected temperature for a pre-selected sterilisation period;
discharging the steam from the chamber; and
a drying method according to the invention.

The pre-conditioning method of the invention has been found to be effective as a pre-conditioning stage for a sterilisation cycle for sterilising a porous load which is a load with more complex geometries (for example, a load comprising an instrument with an internal lumen), a porous load or a load having porous packaging.

The reason why the pre-conditioning method of the invention is effective in pre-conditioning a porous load is believed to be that the holding period allows heat to diffuse thoroughly into the porous load, and also allows steam to diffuse into the porous load due to concentration gradients. Indeed, development work has shown that this holding period to be critical to passing the 'helix test' which tests the effectiveness with which the sterilisation cycle causes steam to penetrate the most challenging loads such as a porous load or a load with a contorted lumen. This test is performed by running a sterilisation cycle with a helix test kit which comprises a coiled lumen having a water-sensitive indicator at its far end. The test demonstrates the ability of the autoclave to sterilise a load having a complex geometry.

The use of the holding period to thoroughly heat the load prior to the second vacuum pulse means that during a subsequent sterilisation stage of a sterilisation cycle, when the chamber is pressurised in order to reach a sterilisation plateau, the interior surfaces of the load are hotter than the steam saturation temperature. This prevents condensation build up on the interior walls of the load, and allows steam to diffuse freely into interior spaces.

Where the chamber of the autoclave used in the invention preferably has an external wall and the autoclave preferably has a heater to heat the external wall of the chamber, the pre-conditioning method of the invention preferably comprises the step of:
heating the external wall of the chamber. This step of the preconditioning method of the invention is preferably carried out either before or immediately after the step of applying a first vacuum pulse in the chamber.

Preferably the pre-conditioning method of the invention consists essentially of the steps defined above. Where the pre-conditioning method of the invention consists essentially of the defined steps, there is a further reason why the pre-conditioning method of the invention is quicker. This is because conventionally more than two vacuum pulses are applied. Typically five or six steps of applying a vacuum pulse and then flushing the chamber with steam are used.

The drying method according to the invention is quicker than known methods when it is run on a chamber having no load or a small load. This is because known drying methods involve applying a vacuum pulse for a fixed period of time. Where a chamber has no load or a small load, a deeper vacuum than is necessary is applied. Also, where the chamber has a full load or a complex porous load, the drying method of the invention is more effective.

As a result of the combination of the use of the pre-conditioning method of the invention and the drying method of the invention, the sterilisation method of the invention is quicker than known methods of performing a sterilisation cycle. The sterilisation method of the invention takes from 41 to 46 minutes with a full load (including a drying stage). Known autoclaves typically take from 50 to 60 minutes.

In the pre-conditioning method of the invention, the length of the holding period depends upon the volume of the chamber of the autoclave. It may be in the range of approximately from 1 to 10 minutes, preferably from 1 to 5 minutes. Where the chamber has a volume of about 10 litres, the length of the holding period is preferably about 6 minutes. Where the chamber has a volume of about 20 litres, the length of the holding period is preferably about 2 minutes. The inventors surmise that the length of the holding period is longer for a smaller chamber is because with a smaller chamber, the other steps of the pre-conditioning method of the invention take less time to perform. Therefore, more time is required during the steam supply step in order for the load to be heated to a sufficient temperature such that steam does not condense on the load during a sterilisation stage of a sterilisation cycle.

The supply of steam is preferably provided by an internal water boiler. An internal water boiler is a water boiler inside the chamber. Such a water boiler is generally provided in a lower part of the chamber. It may have an internal or an external electrical heating element to boil the water. Where the supply of steam is provided by an internal water boiler, the step of supplying steam to the chamber in the pre-conditioning method of the invention preferably comprises the steps of
supplying water to the internal water boiler;
heating the water to supply steam to the chamber at a pressure above atmospheric pressure; and
maintaining the steam pressure in the chamber above atmospheric pressure for a period of time sufficient to heat the contents of the chamber.

A further advantage of the pre-conditioning method of the invention is that it provides a method of performing a pre-conditioning stage with an autoclave with an internal boiler which does not require the use of a large volume of water. The best practice is to use high quality water in an autoclave. This water should ideally be sterile water for inhalation BP or sterile water for injection BP. Such water is very expensive and generally costs around £18/litre. Typically, a known autoclave with an internal water boiler uses from 500 to 1000m1 per cycle. A known autoclave with an external water boiler uses from 200 to 400ml per cycle. A sterilisation cycle using the pre-conditioning stage according to the invention uses at most about 350ml, preferably at most about 250ml.

Because repeated vacuum pulses are not required, the method of the invention is more water efficient. With an internal boiler, using multiple vacuum pulses is a slow process since the water in the boiler continues to generate steam as the pressure drops even if the boiler heater is off. This means the pump has a lot of volume to dispose of in order to reach the required depth of vacuum.

In the preferred method of supplying steam to the chamber, the water is optionally supplied to the internal water boiler when the pressure in the chamber is below atmospheric pressure. This is because the water is then drawn into the chamber by the pressure differential. The step of maintaining the steam pressure optionally involves cycling the water boiler by switching it on or off according to the steam pressure in the chamber.

The vacuum pulse used in the drying method of the invention may reduce the pressure in the chamber to about 400mbarA, preferably to about 300mbarA, and more preferably to about 200mbarA. The steps of the drying method of the invention may be repeated at least two times, preferably at or more than three times. The air supply step of the drying method of the invention may be performed for a pre-selected period of time. Preferably the pre-selected period of time for the air supply step ranges from approximately 5 to 30 seconds, preferably from 10 to 20 seconds, most preferably at about 20 seconds.

In the sterilisation method according to the invention, the chamber is preferably pressurised to a pressure above atmospheric pressure in the step of pressurising the chamber to a sterilisation plateau by supplying steam to the chamber. During the maintaining step of the sterilisation method, the pre-selected temperature and pre-selected sterilisation period are determined by standard sterilisation criteria. The international standard requires a normal procedure of sterilising at 134°C for at least 3 minutes. Where this temperature is not suitable, an alternative procedure of sterilising at 121°C for at least about 15 minutes may also be used.

Preferably the steps of the pre-conditioning, sterilisation and/or drying methods of the invention are carried out sequentially. Where the steps of the pre-conditioning method of the invention are carried out sequentially, the heating step is optionally carried out before, after or at substantially the same time as the first vacuum pulse.

It will be understood that for the sterilisation stage of a sterilisation cycle to be effective, the chamber of the autoclave must be sound. Generally the chamber will have a number of openings such as a door, an air inlet, an outlet and one or more sensor ports. Each of these openings is provided with a seal to prevent air ingress. The seals used to seal the chamber must be sound; they must prevent air ingress. If there is any leakage of air into the chamber, the sterilisation cycle may not sterilise the contents of the chamber.

In order to determine whether a sterilisation cycle is successful, most autoclaves have an air ingress detector connected to the chamber. This detector has a volume for receiving a sample of the contents of the chamber. The detector has a thermocouple for detecting the temperature at the top of the volume of the detector. When the sample of the contents of the chamber comprises only steam, the thermocouple detects a high temperature. When the sample of the contents of the chamber comprises air and steam, the air separates from the steam in the volume of the detector. Heat energy radiates away from the detector, causing the steam to condense and leaving any air trapped at the top of the detector. When sufficient air becomes trapped, steam no longer can condense on the thermocouple, manifesting in a drop in detected temperature. This in turn is the means by which air ingress into the chamber of the autoclave is detected.

A problem with the known air ingress detector is that thermocouples are erratic in their performance and require frequent recalibration. A further problem is that a toxin could become trapped in the volume of the detector, necessitating regular cleansing of the detector. Also, the known air ingress detector adds to the water consumption of the autoclave. A solution to these problems has been sought.

According to the invention there is further provided an autoclave having a chamber having an outlet connected to a condenser and an air ingress detector connected to the condenser wherein the detector has:
a separator vessel for receiving condensed steam from the condenser which vessel is substantially filled with water in use,
a detection tube connected to the vessel which tube is for receiving air separated from the condensed steam, and
a sensor for detecting when the level of water in the tube falls below a pre-determined height to indicate failure of a sterilisation cycle due to ingress of air.

The advantage of the autoclave of the invention is that it has a detector which uses a more reliable sensor to detect the ingress of a sufficient amount of air for a sterilisation cycle to fail. This sensor does not require the difficult calibration of the thermocouple and is more robust. Further advantages include that the problems of trapped toxins and higher water consumption does not arise because the detector is down stream of the condenser. Also the detector is more likely to detect failure of a sterilisation cycle because it checks for the presence of air in substantially all of the condensed steam from the condenser (the thermocouple type detector only samples a small fraction of the steam in the chamber and may miss air trapped in the load, for example).

Preferably in the autoclave of the invention, the detector is connected to a waste water tank. More preferably, the detector is connected to the waste water tank by a restrictor which is arranged so that the pressure in the separation vessel is substantially the same as the pressure in the chamber.

The invention will now be illustrated by way of Example without limiting the scope of the claims by reference to the following Figures of the drawings:
**Figure 1** shows a schematic cross-sectional view of an autoclave with an internal boiler for use in the pre-conditioning, drying and/or sterilisation method of the invention;
**Figure 2** shows a schematic cross-sectional view of an autoclave with an external boiler for use in the pre-conditioning, drying and/or sterilisation method of the invention;
**Figure 3** shows a schematic view of an autoclave according to the invention comprising an air ingress detector; and
**Figure 4** shows a schematic cross-sectional view of the air ingress detector used in the autoclave according to the invention.

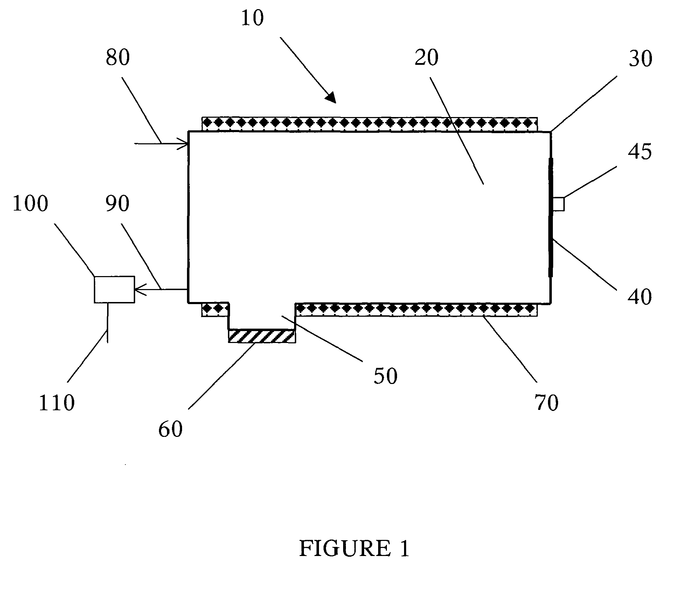

The autoclave 10 shown in Figure 1 has a chamber 20 defined by an external wall 30 and a door 40. The volume of chamber 20 is 22 litres. Door 40 to the chamber 20 has a handle 45 for opening and closing the door 40. The external wall 30 has an electrically-powered wall heater 70. Formed in the base of the chamber 20 is an internal water boiler 50 having an external electrical element 60 for heating water (not shown) in the water boiler 50. The internal water boiler 50 has a water inlet (not shown). The chamber 20 has an air inlet 80 and an outlet 90. Outlet 90 is connected to vacuum pump 100 which has a drain 110 which is connected to a condenser (not shown).

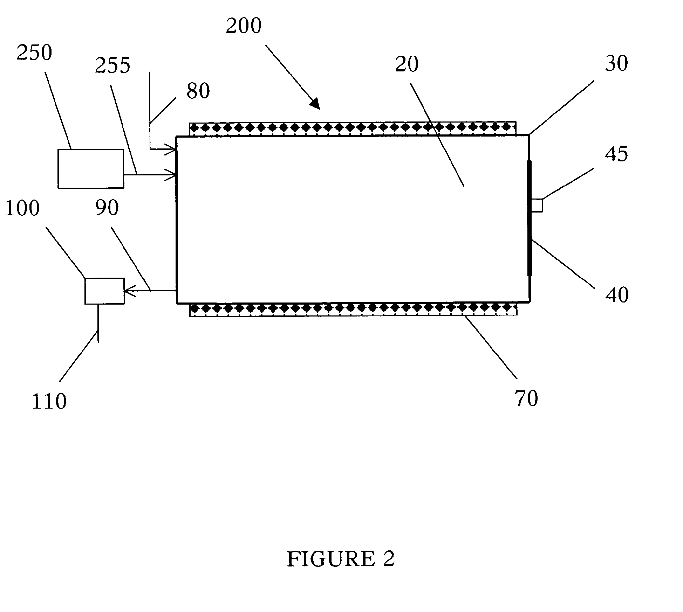

Like features of the autoclave 200 shown in Figure 2 to the autoclave 10 shown in Figure 1 are identified by like reference numerals. Autoclave 200 has a chamber 20 defined by an external wall 30 and a door 40. The volume of chamber 20 is 22 litres. Door 40 to the chamber 20 has a handle 45 for opening and closing the door 40. The external wall 30 has an electrically-powered wall heater 70. The chamber 20 has a steam inlet 255 which is connected to external water boiler 250. The external water boiler 250 has a water inlet (not shown). The chamber 20 has an air inlet 80 and an outlet 90. Outlet 90 is connected to vacuum pump 100 which has a drain 110 which is connected to a condenser (not shown).

As an alternative embodiment to the autoclaves 10,200 shown in Figures 1 and 2, respectively, the volume of the chamber 20 may be 11 litres. As an alternative embodiment to the autoclave 10 shown in Figure 1, the internal water boiler 50 may have an internal electrical element for heating water.

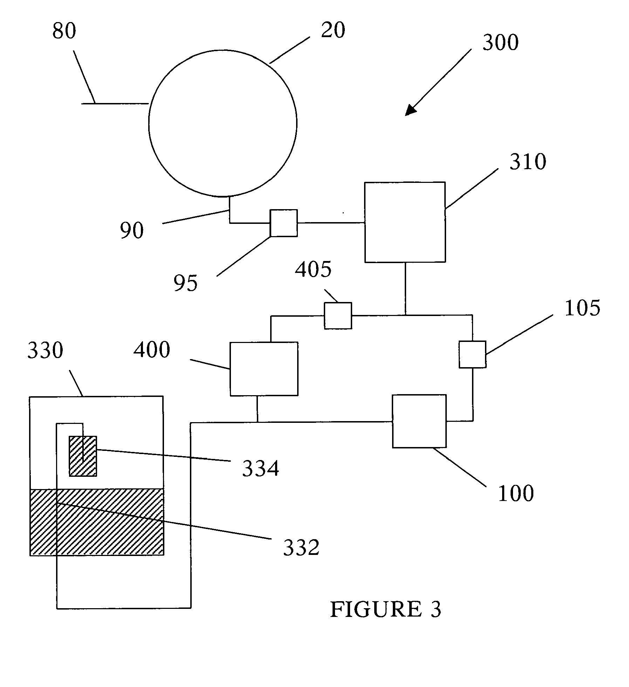

The autoclave 300 shown in Figure 3 has a chamber 20 having an air inlet 80 and an outlet 90. Autoclave 300 also has an internal water boiler (not shown) for providing steam to chamber 20. Outlet 90 is connected via valve 95 to condenser 310. Condenser 310 is connected to vacuum pump 100 via valve 105 and to air ingress detector 400 via valve 405. Both vacuum pump 100 and air ingress detector 400 are connected to waste reservoir 330. Waste reservoir 330 has an inlet 332 which is connected to overflow reservoir 334 which is contained within waste reservoir 330. As an alternative, autoclave 300 may have an external water boiler.

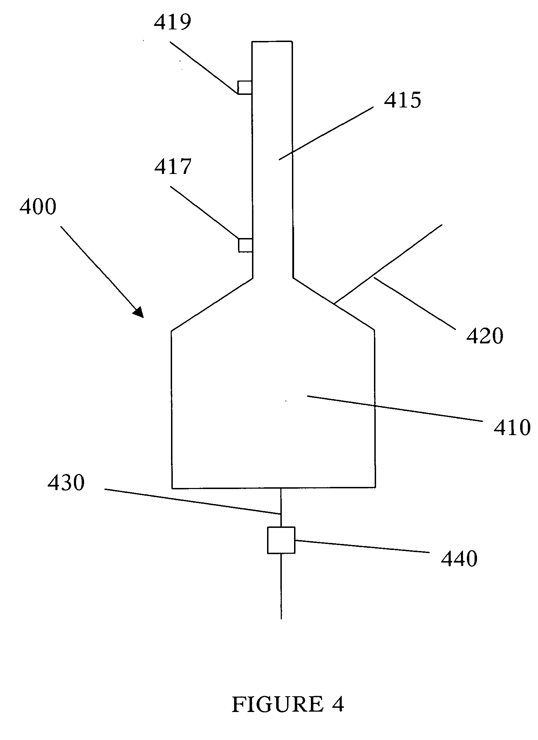

The air ingress detector 400 is shown in detail in Figure 4. Air ingress detector 400 has a separation vessel 410 and detection tube 415. Detection tube 415 is provided with a sensor 417 for sensing the level of water in the detection tube 415. The separation vessel 410 of air ingress detector 400 is connected to valve 405 (not shown in Figure 4) via inlet 420. Separation vessel 410 has an outlet 430 which is connected to restrictor 440. The volume of overflow reservoir 334 (shown in Figure 3) is slightly more than the combined volumes of separation vessel 410 and detection tube 415. Restrictor 440 is connected to waste outlet 330 (not shown in Figure 4) . Restrictor 440 has a diameter sufficiently narrow that the pressure in separation vessel 410 is substantially the same as the pressure in chamber 20 without being so narrow that flow of water through the restrictor is restricted so much that the time taken for the condenser to drain is too long to be acceptable to a user of the autoclave.

In use, the autoclave 300 operates as follows. At the beginning of a sterilisation cycle, overflow reservoir 334 is full from the discharge from the previous cycle. A filling vacuum is drawn in chamber 20 using vacuum pump 100 whilst air inlet 80 is closed, outlet 90 is open, valve 405 is closed and valve 105 is open. Valve 105 is closed and valve 405 is opened to prime detector 400. At this stage, the pressure in the air ingress detector is at a priming pressure which is below atmospheric pressure. This pressure difference causes water in overflow reservoir 334 to siphon into separation vessel 410 via restrictor 440 such that separation vessel 410 fills with water. Valve 405 is opened for one second and is then closed. If water has not reached sensor 419, it is opened again for one second. This process is repeated until sensor 419 detects water such that detector 400 is full of water. Restrictor 440 ensures the air in separation vessel 410 is held at a low pressure while it fills with water. The column of air in detection tube 415 shortens as the pressure rises. If detector 400 is already full of water from a previous cycle and detection tube 415 contains air from a previous cycle, during the priming of detector 400, detection tube 415 is emptied of excess air due to the priming pressure being applied to detector 400. After this excess air has been removed, the detector is re-filled with water as set out above.

The sterilisation cycle is then performed as usual. At the end of the sterilisation stage, steam is discharged from chamber 20 by opening air inlet 80 and outlet 90. Initially valve 105 is opened and valve 405 is closed. This causes air in condenser 310 to be ejected from autoclave 300 such that autoclave 300 is purged of air.

When water ejection from chamber 20 is complete, valve 105 is closed and valve 405 is opened. Steam condensed by condenser 310 is then diverted into air ingress detector 400. Any air which is present in the steam separates from the steam in separation vessel 410 and is trapped in detection tube 415. Initially the trapped air in detection tube 415 is compressed due to the pressure difference between the chamber discharge pressure and atmospheric pressure. As this pressure difference falls, trapped air in detection tube 415 expands such that the level of water in detection tube 415 falls. Valve 405 is closed just before the pressure in chamber 20 reaches atmospheric pressure. Valve 105 is opened and the residue of condensed steam is diverted to waste outlet 330 via vacuum pump 100.

If when the trapped air expands, the level of water in detection tube falls below sensor 417, sufficient air has been trapped in the steam for the cycle to fail. Sensor 417 detects that the level of water has fallen to this extent and a user of autoclave 300 is informed that the sterilisation cycle failed.

The size of separation vessel 410 and detection tube 415 and the position of sensor 417 will depend upon the priming pressure and how much trapped air needs to be present before a sterilisation cycle is deemed to have failed. A person of skill in the art is able to determine these parameters.

The detector may be tested by using an inadequate filling vacuum such that separation vessel 410 does not fill with water from the overflow reservoir 334. This results in excess air in the detector such that the level of water in detection tube 415 is insufficient to reach sensor 417.

The invention is illustrated by reference to the following Examples which are not intended to limit the scope of the claimed invention.

### EXAMPLE 1

In this Example, a vacuum sterilisation cycle according to the invention was performed on the autoclave 10 shown in Figure 1. The vacuum sterilisation cycle had three stages: pre-conditioning, sterilisation and drying stages. The chamber wall heater 20 was operated between cycles.

### PRE-CONDITIONING STAGE

The steps of the preconditioning stage of the vacuum sterilisation cycle were as follows:
1. A vacuum pulse was applied by opening outlet 90 and operating vacuum pump 100; the wall heater 70 was applied to heat the external wall 30 in steps 1-8 inclusive;
2. The internal boiler 50 was filled with water;
3. The internal boiler 50 was operated to heat the water to generate steam in chamber 20 until the chamber pressure was above atmospheric;
4. The chamber pressure was held above atmospheric for 2 minutes by cycling the internal boiler 50 by switching it on and off;
5. Steam in chamber 20 was vented;
6. A vacuum pulse was applied by opening outlet 90 and operating vacuum pump 100; and
7. Internal boiler 50 was operated to heat the water to generate steam in chamber 20 to start pressurising in preparation for the sterilisation stage which is carried out at a pressure of 2.16barA.

### STERILISATION STAGE

The sterilisation stage comprised the following steps:
8. Chamber 20 was heated to a sterilisation plateau of 134°C;
9. The temperature in chamber 20 was held at 134°C for a sterilisation period of 3 minutes; during this stage, the wall heater was off; and
10. Steam and residual water was discharged from chamber 20 ; the wall heater 70 was applied to heat the external wall 30 of chamber 20.

### DRYING STAGE

The drying stage comprised the following steps:
11. A vacuum pulse was applied by opening outlet 90 and operating vacuum pump 100 to reduce the pressure in chamber 20;
12. Filtered air was allowed to flow into chamber 20 via air inlet 80 for 20 seconds (causing the pressure to rise again);
13. Steps 11 and 12 were repeated five times; with the 20 second air inlet between each pulse;
14. Air inlet 80 was opened to allow air into the chamber until atmospheric pressure was reached; and
15. Air inlet 80 and outlet 90 were opened and closed as necessary to maintain atmospheric pressure until the user had extracted the load (not shown) from chamber 20 via door 40.

The vacuum sterilisation cycle set out above passed the Helix test.

### EXAMPLE 2

In this Example, a vacuum sterilisation cycle according to the invention was performed on the autoclave 10 shown in Figure 1 having a chamber volume of 11 litres. The vacuum sterilisation cycle had three stages: pre-conditioning, sterilisation and drying stages. The chamber wall heater 20 was operated between cycles.

### PRE-CONDITIONING STAGE

The steps of the preconditioning stage of the vacuum sterilisation cycle were as follows:
1. A vacuum pulse was applied by opening outlet 90 and operating vacuum pump 100 to reduce the pressure in chamber 20; the wall heater 70 was applied to heat the external wall 30 during steps 2-8 inclusive;
2. The internal boiler 50 was filled with water;
3. The internal boiler 50 was operated to heat the water to generate steam in chamber 20;
4. The chamber pressure was held for 6 minutes by cycling the internal boiler 50 by switching it on and off;
5. Steam in chamber 20 was vented to reduce the chamber pressure;
6. A vacuum pulse was applied by opening outlet 90 and operating vacuum pump 100 to reduce the pressure in chamber 20; and
7. Internal boiler 50 was operated to heat the water to generate steam in chamber 20 to start pressurising in preparation for the sterilisation stage which is carried out at a pressure of 2.16barA.

### STERILISATION STAGE

The sterilisation stage comprised the following steps:
8. Chamber 20 was heated to a sterilisation plateau of 134°C;
9. The temperature in chamber 20 was held at 134°C for a sterilisation period of 3 minutes; during this stage, the wall heater was off; and
10. Steam and residual water was discharged from chamber 20 and the chamber pressure was reduced; the wall heater 70 was applied to heat the external wall 30 of chamber 20.

### DRYING STAGE

The drying stage comprised the following steps:
11. A vacuum pulse was applied by opening outlet 90 and operating vacuum pump 100 to reduce the pressure in chamber 20;
12. Filtered air was allowed to flow into chamber 20 via air inlet 80 for 20 seconds (causing the pressure to rise again);
13. Steps 11 and 12 were repeated eight times;
14. Air inlet 80 was opened to allow air into the chamber until atmospheric pressure was reached;
15. Air inlet 80 and outlet 90 were opened and closed as necessary to maintain atmospheric pressure until the user had extracted the load (not shown) from chamber 20 via door 40.

The vacuum sterilisation cycle set out above passed the Helix test.

### EXAMPLE 3

The vacuum sterilisation cycles of Examples 1 and 2 were performed on an autoclave 200 as shown in Figure 2 having a chamber volume of 22 and 11 litres, respectively. Where a step in the vacuum sterilisation cycle refers to the operation of the internal boiler 50, the external boiler 250 was used and the steam inlet 255 was opened to allow steam generated by the external boiler 250 to enter chamber 20. Each cycle passed the Helix test.

## Claims

1. A pre-conditioning method of operating an autoclave (10) during a pre-conditioning stage of a sterilisation cycle wherein the autoclave (10) has a chamber (20) having an external wall (30), a supply of steam, a heater (70) to heat the external wall (30) of the chamber (20) and a vacuum pump (100) which method comprises the steps of:
applying a first vacuum pulse in the chamber (20);
heating the chamber (20);
supplying steam to the chamber (20) at a pressure above atmospheric pressure for a holding period which is sufficiently long for the contents of the chamber (20) to be heated; and
applying a second vacuum pulse in the chamber (20).

2. A method as defined in Claim 1 wherein the autoclave (10) has a chamber (20) having an external wall (30) and a heater (70) to heat the external wall (30) of the chamber (20) and wherein the method comprises a step of:
heating the external wall (30) of the chamber (20).

3. A method as defined in Claim 1 or Claim 2 wherein the autoclave (10) has a chamber (20) having an external wall (30) and a heater (70) to heat the external wall (30) of the chamber (20) and wherein the method consists essentially of the steps of:
applying a first vacuum pulse in the chamber (20);
heating the external wall (30) of the chamber (20);
supplying steam to the chamber (20) at a pressure above atmospheric pressure for a holding period which is sufficiently long for the contents of the chamber (20) to be heated; and
applying a second vacuum pulse in the chamber (20).

4. A method as defined in any one of the preceding Claims wherein the chamber (20) contains a porous load.

5. A method as defined in any one of the preceding Claims wherein the supply of steam is provided by an internal water boiler (50).

6. A method as defined in Claim 5 wherein the step of supplying steam to the chamber (20) comprises the steps of:
supplying water to the internal water boiler (50);
heating the water to supply steam to the chamber (20) at a pressure above atmospheric pressure; and
maintaining the steam pressure in the chamber (20) above atmospheric pressure for a period of time sufficient to heat the contents of the chamber (20).

7. A method as defined in any one of the preceding Claims wherein the steps are carried out sequentially.

8. A method of operating an autoclave (10) to perform a sterilisation cycle wherein the autoclave (10) has a chamber (20) having an external wall (30), a supply of steam, a heater (70) to heat the external wall (30) of the chamber (20) and a vacuum pump (100) which method comprises the steps of
a method as defined in any one of Claims 1 to 7;
pressurising the chamber (20) to a sterilisation plateau by supplying steam to the chamber (20);
maintaining the chamber (20) at a pre-selected temperature for a pre-selected sterilisation period;
discharging the steam from the chamber (20); and
a drying stage comprising the steps of:
applying a vacuum pulse until the pressure in the chamber (20) reaches a pre-set level;
supplying the chamber (20) with air.

9. A method as defined in Claim 8 wherein the steps of the drying stage are repeated at least two times.

10. A method as defined in Claim 8 or Claim 9 wherein the air supply step of the drying stage is performed for a pre-selected period of time.

11. A method as defined in any one of Claims 8 to 10 wherein the chamber (20) contains a porous load.

12. A method as defined in any one of Claims 8 to 11 wherein the steps are carried out sequentially.

13. A method as defined in Claim 8 wherein the chamber (20) is pressurised to a pressure above atmospheric pressure in the step of pressurising the chamber (20) to a sterilisation plateau by supplying steam to the chamber (20).
